# EUROPÄISCHE PATENTSCHRIFT

(11) **EP 1 526 827 B1**
(45) Veröffentlichungstag und Bekanntmachung des Hinweises auf die Patenterteilung: **09.12.2009**
(21) Anmeldenummer: 03740332.6
(22) Anmeldetag: 25.06.2003
(51) Int. Cl.: A61K 8/06, A61K 8/891, A61Q 15/00

(54) **KOSMETSCHE UND/ODER DERMATOLOGISCHE ZUBEREITUNG**
COSMETIC AND/OR DERMATOLOGICAL PREPARATION
PREPARATION COSMETIQUE ET/OU DERMATOLOGIQUE

(30) Priorität: 31.07.2002 DE 10234883
(43) Veröffentlichungstag der Anmeldung: 04.05.2005
(73) Patentinhaber: BEIESRDORF AG, 20245 Hamburg (DE)
(72) Erfinder: DIEC, Khiet, Hien, 20251 Hamburg (DE); KUX, Ulrich, 22559 Hamburg (DE)
(86) Internationale Anmeldenummer: PCT/EP2003/006678
(87) Internationale Veröffentlichungsnummer: WO 2004/014324

(56) Entgegenhaltungen:
- EP-A- 1 125 574
- WO-A-01/28510
- US-A- 5 635 165

## Beschreibung

Die vorliegende Erfindung betrifft kosmetische und/oder dermatologische Antitranspirantien-Zubereitungen in Form von Emulsionen, die bestimmte Silikonemulgatoren enthalten.

Zur Veränderung des Körpergeruchs des Menschen gibt es mehrere Wege. Zum einem kann durch Parfums oder ähnliche Zubereitungen der Eigengeruch des Körpers überdeckt werden. Eine andere Möglichkeit ist es, die Bildung bestimmter Formen des Eigengeruches des Körpers zu verhindern. Eine von einigen Menschen als unangenehm empfundene und daher von vielen Menschen unerwünschte Form des körpereigenen Geruchs ist der Schweißgeruch. Obwohl der vom Körper abgegebene Schweiß nahezu geruchlos ist, entsteht durch bakterielle Zersetzung desselben jene zuweilen als unerwünscht angesehene Duftnote. Um das Auftreten des so entstehenden Körpergeruches zu verhindern oder zu verringern, können unter anderem Deodorant- und Antitranspirantzubereitungen eingesetzt werden. Deodorantien verhindern, daß auf der Haut siedelnde Bakterien den Schweiß zersetzen, indem sie in ihrer Aktivität oder ihrem Wachstum gehemmt oder abgetötet werden. Antitranspirantien hingegen verhindern oder verringern die Schweißabsonderung an sich und entziehen den geruchsverursachenden Bakterien so ihr Betätigungsfeld.

Üblicherweise werden Antitranspirantien in mannigfaltigen Produktformen angeboten, wobei in Europa Roller, Pumpzerstäuber und Aerosole dominieren, in den USA, Mittel- und Südamerika Stifte. Es sind sowohl wasserfreie als auch wasserhaltige Produkte (hydro-alkoholische Formulierungen, Emulsionen) bekannt. Das grundsätzliche Problem bei Antitranspirantien enthaltenden Emulsionen besteht in einem destabilisierenden Effekt hoher Gehalte an Elektrolyten, insbesondere bei Zubereitungen mit niedrigen pH-Werten. Dadurch sind Emulsionen nicht immer lagerstabil und unterliegen oft Erscheinungen wie Aufrahmung oder Sedimentation. Dies gilt insbesondere für dünnflüssige Emulsionen mit niedriger Viskosität.

Gleichzeitig müssen derartige Zubereitungen vom Anwender als angenehm anwendbar empfunden werden.

Emulgatoren auf der Basis von Silikonen an sich sind bereits bekannt.

Die europäische Patentanmeldung 1 125 574 beschreibt beispielsweise O/W-Emulsionen, die bestimmte lineare Polyethersiloxane als Emulgatoren enthalten.

Verwendet man in Antitranspirantien enthaltenden Zubereitungen derartige Emulgatoren, so werden zumeist keine langzeitlagerstabilen Präparate erhalten.

Die Gesellschaft Goldschmidt AG bietet unter der CTFA-Bezeichnung Bis-PEG/PPG-16/16 PEG/PPG-16/16 Dimethicone (and) Caprylic/Capric Triglycerid einen silikonbasierten nichtionischen Emulgator für O/W-Emulsionen an, bei dem es sich um eine klare Flüssigkeit mit einem HLB-Wert von etwa 10 handelt. Der Handelsname lautet Abil Care 85. Chemisch besteht es aus einer Mischung der Ester aus Glycerin und Capryl- und/oder Caprinsäure, die keine unveresterten Hydroxylgruppen aufweisen:

ROCH₂-ROCH-CH₂OR;

wobei R CH₃(CH₂)₆CO- und/oder CH₃(CH₂)₈CO- bedeutet,
linearen Polydimethylsiloxanen,

R²-(Si(CH₃)₂O)ₙ₋₁₋ₘ-(Si(CH₃)R²O)ₘ-Si(CH₃)₂-R²

die mit zwei oder drei Resten R² substituiert sind Dabei sind immer zwei Reste R² durch eine Kohlenstoff-Silicium-Bindung mit den beiden terminalen Siliciumatomen verbunden und der gegebenenfalls dritte Rest R² mit einem dem Siloxankettenende benachbarten Siliciumatom. R² besteht dabei aus einer kurzen Alkylkette, an die k Ethylenoxyeinheiten und darauf folgend I Propylen-2-oxyeinheiten gebunden sind, an deren Ende eine freie Hydroxylgruppe folgt:

-(CH₂)ᵢ-(O-CH₂-CH₂)ₖ-(O-C₃H₆)ₗ-OH

Dabei betragen im Mittel i etwa 1 bis 3, k etwa 16, I etwa 16, n etwa 80. Der Wert von m beträgt entweder 0 oder 1, wobei die sich dabei ergebenden Polydimethylsiloxane etwa im Verhältnis 1:1 stehen.

Verwendet man in Antitranspirantien enthaltenden Zubereitungen derartige Emulgatoren, so konnten bisher keine langzeitlagerstabilen Präparate erhalten werden.

Ausgehend hiervon stellte sich die Aufgabe, ein Antitranspirantien enthaltendes Präparat zu finden, das beim Auftragen auf die Haut ein angenehmes Gefühl hinterlässt und auch bei niedrigen pH-Werten und hohen Gehalten an Elektrolyten stabil bleibt.

Es hat sich für den Fachmann nicht vorhersehbar herausgestellt, daß kosmetische und/oder dermatologische Zubereitungen in Form von Emulsionen, besonders bevorzugt Öl-in-Wasser-Emulsionen, **dadurch gekennzeichnet, daß**
(a) sie Antitranspirantien enthalten,
(b) lineare Polydimethylsiloxane R²-(Si(CH₃)₂O)ₙ₋₁-Si(CH₃)₂-R², enthalten sind,
(c) Polydimethylsiloxane R²-(Si(CH₃)₂O)ₙ₋₂-Si(CH₃)R²O-Si(CH₃)₂-R², enthalten sind,
(d) wobei
   (d1) R² die Gruppierung -(CH₂)ᵢ-(O-CH₂-CH₂)ₖ-(O-C₃H₆)ₗ-OH repräsentiert,
   (d2) wobei i 2 bis 4 , k und I 12 bis 20 betragen,
   (d3) n Werte von 60-100 aufweist und
   (d4) die Polydimethylsiloxane (b) und (c) im Verhältnis 80:120 bis 120:80 stehen und
(e) der Anteil der Emulgatoren weniger als 10 Gew.%, besonders bevorzugt weniger als 8 Gew.%, ganz besonders bevorzugt 5 Gew.% beträgt,
den Mängeln des Standes der Technik abhelfen.

Es wurde weiter gefunden, daß es bevorzugt ist, wenn solche Zubereitungen einen Gehalt an nichtionischen Coemulgatoren gewählt aus der Gruppe der Fettalkoholethoxylate oder Gemischen derselbigen, wobei im Falle der Verwendung von Fettalkoholethoxylat-Gemischen eine Komponente eine Kettenlänge von weniger als 14 Kohlenstoffatomen und die andere Komponente eine Kettenlänge von mehr als 16 Kohlenstoffatomen besitzt, bevorzugt Cetylstearylalkohol,
der Fettsäureethoxylate,
der Fettsäuremonoglycerinester, deren Fettsäurekomponente höchstens 24 oder wenigstens 8 Kohlenstoffatome enthält,
der Fettalkohole,
der ethoxlierten Sorbitanester mit einem Ethoxylierungsgrad von 3 bis 100
aufweisen.
Ebenfalls bevorzugt ist es, wenn im Mittel i 3 , k und I 16, n 80 betragen und die Polydimethylsiloxane (b) und (c) im Verhältnis 1:1 stehen. Weiterhin ist es bevorzugt, wenn die Ölphase Ölkomponenten enthält, die aus folgenden Gruppen gewählt werden: Ester aus einwertigen und/oder mehrwertigen Fettalkoholen mit gesättigten und/oder ungesättigten, verzweigten und/oder unverzweigten Alkancarbonsäuren einer Kettenlänge von 3 bis 30, insbesondere 12 bis 25 Kohlenstoffatomen, gesättigten und/oder ungesättigten, verzweigten und/oder unverzweigten Fettalkoholen einer Kettenlänge von 3 bis 30, insbesondere 12 bis 25 Kohlenstoffatomen, cyclischen oder linearen, gesättigten und/oder ungesättigten, verzweigten und/oder unverzweigten Silikonölen, Fettsäure-Triglyceride, namentlich Triglycerinester gesättigter und/oder ungesättigter, verzweigter und/oder unverzweigter Alkansäuren einer Kettenlänge von 8 bis 24, insbesondere 12 bis 18 Atomen, sowie der Mineralöle.
Dabei ist es besonders bevorzugt, wenn der Anteil der Ölkomponenten kleiner als 15 Gew.%, ganz besonders bevorzugt kleiner als 10 Gew.% ist.
Weiterhin ist es besonders bevorzugt, wenn die Zubereitungen einen Stabilisator gewählt aus der Gruppe der Sorbitolpolyoxyethylenderivate und/oder der Betaine enthalten.
Dabei ist es bevorzugt, wenn sie 5 bis 40 Gew.%, besonders bevorzugt 10 bis 25 Gew.% Antitranspirantien enthalten.
Bevorzugt werden solche Zubereitungen verwendet als Mittel zur Hemmung der Schweißabsonderung des Menschen.
Die erfindungsgemäßen Zubereitungen lassen sich besonders leicht applizieren, hinterlassen auf der Haut bei der Anwendung ein angenehmes Hautgefühl, indem sie zunächst durch den Wassergehalt einen frischen, kühlenden Eindruck erzeugen, schnell einziehen, sich nicht fettig anfühlen und dabei insbesondere nicht kleben. Üblicherweise werden zur Einstellung eines solchen Hautgefühls in größerem Umfang Silikonöle eingesetzt. Dies ist hier nicht erforderlich, man erreicht durch Einsatz des erfindungsgemäßen Emulgator/Stabilisatorsystems auch mit nichtsilikonischen Ölen eine Sensorik, wie sie typischerweise nur mit Silikonölen erzielt werden kann. Dies ist speziell für wasserhaltige Rezepturen wie Emulsionen von großer Bedeutung, bei denen die in gelöster Form vorliegenden Antitranspirant-Salze ohne entsprechende formeltechnische Maßnahmen ein vom Verbraucher unerwünschtes klebriges Hautgefühl erzeugen. Darüberhinaus hinterlassen die erwähnten Antitranspirantsalze einen ebenfalls unerwünschten sichtbaren weißen Rückstand auf der Haut, der mit Silikonölen aufgrund derer Leichtflüchtigkeit nicht langanhaltend und effizient kaschiert werden kann.

Daher kann auch der für größere Mengen an Silikonöl enthaltende Zubereitungen typische Effekt der ungenügenden Wirkleistung vermieden werden, da diese bislang in Ermangelung eines geeigneten O/W-Emulgatorsystems als Wasser-in-Silikonöl-Emulsionen formuliert werden mußten, die den Nachteil einer schlechten Wirkfreisetzung des Antitranspirant-Salzes aus der inneren Emulsionsphase (Wasser) sowie ein glitschiges Hautgefühl mit sich bringen.

Vorteilhaft kann dabei die Viskosität im Bereich 50 bis 5000 mPa s, besonders bevorzugt von 100 bis 1500 mPa s, je nach Applikationsanforderung, beliebig eingestellt werden.

Die Gehalte an Antitranspirantien beziehen sich auf die sog. Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Als Aluminium-Salze (der empirischen Summenformel [Al₂(OH)ₘClₙ], wobei m+n=6) können erfindungsgemäß Aluminiumchlorid AlCl₃, Aluminiumsulfat Al₂(SO₄)₃, Aluminiumchlorhydrat [Al₂(OH)₅Cl] • H₂O, Aluminiumsesquichlorhydrat [Al₂(OH)_{4.5}Cl_{1.5}] • H₂O, Aluminiumdichlorhydrat [Al₂(OH)₄Cl₂] • H₂O und auch im Handel erhältliche Komplexe wie Locron L (Clariant), Chlorhydrol (Reheis), ACH-303 (Summit), Aloxicoll L (Giulini), Reach 501 (Reheis), AACH-324 (Summit), Aluminum Sesquichlorohydrate (Reheis), ACH-308 (Summit), Aloxicoll 31 L (Giulini), Reach 301 (Reheis) verwendet werden.

Als Aluminium-Zirkonium-Salze können erfindungsgemäß Aluminium/Zirkonium Trichlorhydrex Glycin [Al₄Zr(OH)₁₃Cl₃] • H₂O • Gly, Aluminium/Zirkonium Tetrachlorhydrex Glycin [Al₄Zr(OH)₁₂Cl₄] • H₂O • Gly, Aluminium/Zirkonium Pentachlorhydrex Glycin [Al₆Zr(OH)₂₃Cl₅] • H₂O • Gly, Aluminium/Zirkonium Octachlorhydrex Glycin [Al₈Zr(OH)₂₀Cl₈]•H₂O • Gly, aber auch Glycin-freie Aluminium/Zirkonium-Salze und insbesondere im Handel erhältliche Komplexe wie Rezal 33GP (Reheis), AZG-7164 (Summit), Zirkonal P3G (Giulini), Reach AZZ 902 (Reheis), AAZG-7160 (Summit), Zirkonal AP3G (Giulini), Rezal 36G (Reheis), AZG-368 (Summit), Zirkonal L435G (Giulini), Reach AZP 855 (Reheis), AAZG-6313-15 (Summit), Zirkonal AP4G (Giulini), Rezal 67 (Reheis), Zirkonal L540 (Giulini), Reach AZN 885 (Reheis) verwendet werden.

Dabei soll die Verwendung der Antitranspirant-Wirker aus den Rohstoffklassen Aluminium- und Aluminium/Zirkonium-Salzen nicht auf die handelsüblichen zumeist wäßrigen Lösungen, wie z.B. Locron L (Clariant), beschränkt sein, sondern es kann auch von Vorteil sein, die ebenfalls handelsüblichen wasserfreien Pulver derselbigen Rohstoffe durch Einbringung in die beanspruchten Formulierungen zum Einsatz zu bringen, wie z.B. Locron P (Clariant).

Vorteilhaft ist auch die Verwendung von sog. AT-Salz Suspensionen sein, bei denen pulverförmig vorliegende Aluminium- und Aluminium/Zirkonium-Salze in diversen Ölen dispergiert angeboten werden.

Desweiteren kann es aber auch von Vorteil sein, spezielle Aluminium- und Aluminium/Zirkonium-Salze zum Einsatz zu bringen, die zur Löslichkeitsverbesserung als Glykol-Komplexe angeboten werden.

Weitere vorteilhafte Antitranspirant-Wirker basieren anstelle von Aluminium bzw. Zirkonium auf anderen Metallen, wie z.B. Beryllium, Titan, Hafnium.

Dabei soll die Liste der verwendbaren Antitranspirant-Wirker aber nicht auf metallhaltige Rohstoffe begrenzt sein, sondern von Vorteil sind auch Verbindungen, die Nichtmetalle wie Bor enthalten sowie solche, die dem Bereich der organischen Chemie zuzurechnen sind, wie z.B. Anticholinergika.

Vorteilhaft können erfindungsgemäßen Zubereitungen Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Liste der genannten Wirkstoffe bzw. Wirkstoffkombinationen, die in den erfindungsgemäßen Emulsionen verwendet werden können, soll selbstverständlich nicht limitierend sein.

Die Menge der Desodorantien (eine oder mehrere Verbindungen) in den Zubereitungen beträgt vorzugsweise 0,01 bis 10 Gew.%, besonders bevorzugt 0,05 bis 5 Gew.%, insbesondere 0,1 bis 1 Gew.% bezogen auf das Gesamtgewicht der Zubereitung.

Sofern als Ölkomponenten nicht die als bevorzugt gekennzeichneten allein zum Einsatz gelangen, können diese zusätzlich oder alternativ gewählt werden aus den Gruppen der polaren Öle, der mittelpolaren Öle, der niederpolaren Öle, der Esteröle, der Wachse und/oder der Silikonöle.
oder
Die Lipidphase der erfindungsgemäßen kosmetischen oder dermatologischen Emulsionen kann vorteilhaft gewählt werden aus den Gruppen der polaren Öle, der mittelpolaren Öle, der niederpolaren Öle, der Esteröle, der Wachse und/oder der Silikonöle.

Als Oberbegriff für Fette, Öle, Wachse und dergleichen wird gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächen-spannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächen-spannung gegen Wasser weniger als 20 mN/m beträgt als unpolar solche, deren Grenzflächenspannung gegen Wasser mehr als 30 mN/m beträgt. Lipide mit einer Grenzflächenspannung gegen Wasser zwischen 20 und 30 mN/m werden im allgemeinen als mittelpolar bezeichnet.

Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkemöl, Distelöl, Nachtkerzenöl, Macadamianußöl, Maiskeimöl, Avocadoöl und dergleichen sowie die mit ihrer INCI-Bezeichnung folgenden
- Butyl Decanol (+) Hexyl Octanol (+) Hexyl Decanol (+) Butyl Octanol, beispielsweise erhältlich unter der Bezeichnung Isofol 14 T von der Gesellschaft Condea Chemie,
- Tridecyl Stearate(+) Tridecyl Trimellitate(+) Dipentaerythrityl Hexacaprylate/Hexacaprate, beispielsweise erhältlich unter der Bezeichnung Lipovol MOS-130 von der Gesellschaft Lipochemicals INC,
- Propylene Glycol Dicaprylate/Dicaprate, beispielsweise erhältlich unter der Bezeichnung Miglyol 840 von der Gesellschaft CONDEA Chemie,
- Butyl Octanol, beispielsweise erhältlich unter der Bezeichnung Isofol 12 von der Gesellschaft CONDEA Chemie,
- Stearyl Heptanoate, beispielsweise erhältlich unter der Bezeichnung Tegosoft SH von der Gesellschaft Goldschmidt,
- Dibutyl Adipate, beispielsweise erhältlich unter der Bezeichnung Cetiol B von der Gesellschaft Cognis,
- PEG 2 Diethylenhexanoate, beispielsweise erhältlich unter der Bezeichnung Dermol 488 von der Gesellschaft ALZO (ROVI),
- C12-13 Alkyl Lactate, beispielsweise erhältlich unter der Bezeichnung Cosmacol ELI von der Gesellschaft Condea Augusta,
- Diethylen Glycol Dioctanoate/ Diisononanoate, beispielsweise erhältlich unter der Bezeichnung Dermol 489 von der Gesellschaft ALZO (ROVI),
- Di-C12/13 Alkyl Tartrate, beispielsweise erhältlich unter der Bezeichnung Cosmacol ETI von der Gesellschaft Condea Augusta,
- Propylene Glycol Monoisostearate, beispielsweise erhältlich unter der Bezeichnung Emerest 2384 von der Gesellschaft Cognis,
- Cocoglycerides, beispielsweise erhältlich unter der Bezeichnung Myritol 331 von der Gesellschaft Cognis,
- Triisostearin, beispielsweise erhältlich unter der Bezeichnung Prisorine 2041 GTIS von der Gesellschaft Unichema,
ebenso die Gruppe der Dialkylether, der Gruppe der gesättigten oder ungesättigten, verzweigten oder unverzweigten Alkohole, wobei es insbesondere vorteilhaft ist, wenn die Ölphase einen Gehalt an C₁₂₋₁₅-Alkylbenzoat aufweist oder vollständig aus diesem besteht, die Gruppe der Guerbetalkohole, die selbst bei niederen Temperaturen flüssig sind und praktisch keine Hautreizungen bewirken und die vorteilhaft sie als fettende, überfettende und auch rückfettend wirkende Bestandteile in Haut- und Haarpflegemitteln eingesetzt werden können und die meist durch durch die Struktur 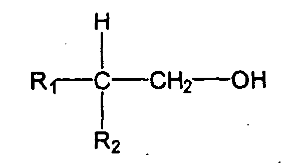 gekennzeichnet sind, wobei R₁ und R₂ in der Regel unverzweigte Alkylreste bedeuten und die Guerbet-Alkohole vorteilhaft gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl darstellt, bevorzugt 2-Butyloctanol, beispielsweise unter der Handelsbezeichnung Isofol^{®} 12 von der Gesellschaft Condea Chemie GmbH erhältlich, das 2-Hexyldecanol, beispielsweise unter der Handelsbezeichnung Isofol® 16 von der Gesellschaft Condea Chemie GmbH erhältlich, wobei auch Mischungen von erfindungsgemäßen Guerbet-Alkoholen sind erfindungsgemäß vorteilhaft zu verwenden sind wie beispielsweise Mischungen aus 2-Butyloctanol und 2-Hexyldecanol wie sie unter der Handelsbezeichnung Isofol® 14 von der Gesellschaft Condea Chemie GmbH erhältlich sind; dabei beträgt die Gesamtmenge anGuerbet-Alkoholen in den fertigen kosmetischen oder dermatologischen Zubereitungen wird vorteilhaft aus dem

Bereich bis 25,0 Gew.-%, bevorzugt 0,5 - 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Lipide im Sinne der vorliegenden Erfindung sind die im folgenden mit ihrer INCI-Bezeichnung aufgelisteten Substanzen:
- Isodecyl Neopentanoate, beispielsweise erhältlich unter der Bezeichnung DUB VCI 10 von der Gesellschaft Stearinerie Dubois Fils,
- Isohexyldecanoate, beispielsweise erhältlich unter der Bezeichnung Dermol IHD von der Gesellschaft ALZO (ROVI),
- Isodecyl Octanoate, beispielsweise erhältlich unter der Bezeichnung Dermol 108 von der Gesellschaft ALZO (ROVI),
- Dihexyl Ether,
- Isodecyl 3,5,5 Trimethyl Hexanoate, beispielsweise erhältlich unter der Bezeichnung Dermol 109 von der Gesellschaft ALZO (ROVI),
- Cetearyl Isononanoate, beispielsweise erhältlich unter der Bezeichnung Cetiol SN von der Gesellschaft Henkel Cognis,
- Isopropylpalmitat, beispielsweise erhältlich unter der Bezeichnung Isopropylpalmitat von der Gesellschaft Unichema,
- Cyclomethicone, beispielsweise erhältlich unter der Bezeichnung DC Fluid 345 von der Gesellschaft Dow Corning,
- Cyclopolydimethylsiloxan, beispielsweise erhältlich unter der Bezeichnung Dow Corning Fluid 244 von der Gesellschaft Dow Corning,
- Dimethicone, beispielsweise erhältlich unter der Bezeichnung Wacker AK 100 von der Gesellschaft Wacker,
- 2- Ethylhexanosäure 3,5,5 Trimethylester, beispielsweise erhältlich unter der Bezeichnung Dermol 98 von der Gesellschaft ALZO (ROVI),
- Octyldodecanol, beispielsweise erhältlich unter der Bezeichnung Eutanol G von der Gesellschaft Henkel Cognis,
- Hexyl Decanol, beispielsweise erhältlich unter der Bezeichnung Isofol 16 von der Gesellschaft Condea Chemie,
- Isotridecyl 3,5,5 Trimethylhexanonanoate, beispielsweise erhältlich unter der Bezeichnung Dermol 139 von der Gesellschaft ALZO (ROVI),
- Hexyldecanol (+) Hexyl Decyl Laurate, beispielsweise erhältlich unter der Bezeichnung Cetiol PGL von der Gesellschaft Henkel Cognis,
- Octyl Palmitate, beispielsweise erhältlich unter der Bezeichnung Cegesoft C24 von der Gesellschaft,
- Octyldodeceyl Myristate, beispielsweise erhältlich unter der Bezeichnung M.O.D. von der Gesellschaft Gattefossé,
- Phenyl Trimethicone, beispielsweise erhältlich unter der Bezeichnung Silikonöl VP 1120 von der Gesellschaft Dow Corning,
- Butyl Octanoicacid, beispielsweise erhältlich unter der Bezeichnung Isocarb 12 von der Gesellschaft CONDEA Chemie,
- Isopropyl Stearate, beispielsweise erhältlich unter der Bezeichnung Isopropylstearat von der Gesellschaft Henkel Cognis,
- C12-15 Alkyl Benzoate, beispielsweise erhältlich unter der Bezeichnung Finsolv TN von der Gesellschaft Goldschmidt,
- Butylene Glycol Caprylate/Caprate, beispielsweise erhältlich unter der Bezeichnung Dermofeel BGC von der Gesellschaft Dr. Straetmans,
- Caprylic/Capric Triglyceride, beispielsweise erhältlich unter der Bezeichnung Miglyol 812 von der Gesellschaft Huels,
- Tricaprylin, beispielsweise erhältlich unter der Bezeichnung Trivent OCG von der Gesellschaft Trivent,
- PEG " Diethylhexanoate/ Diisononanoate/ Ethylhexyl Isononanoate, beispielsweise erhältlich unter der Bezeichnung Dermol 866 von der Gesellschaft ALZO (ROVI).
Niederpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte niederpolare Lipide im Sinne der vorliegenden Erfindung sind die im folgenden mit ihrer INCI- oder Handelsbezeichnung aufgelisteten Substanzen:
- Cycloparaffin, beispielsweise erhältlich unter der Bezeichnung Ecolane 130 von der Gesellschaft Total SA,
- Polydecene von der Gesellschaft Nexbase 2006 FG,
- Hydrogenated Polyisobutene, beispielsweise erhältlich unter der Bezeichnung Polysynlane von der Gesellschaft Chemische Fabrik Lehrte,
- Polydimethylsiloxan, beispielsweise erhältlich unter der Bezeichnung Wacker Silikonöl AK 50 von der Gesellschaft Wacker,
- Isohexadecane, beispielsweise erhältlich unter der Bezeichnung Solvent ICH von der Gesellschaft EC Erdölchemie,
- Mineral Oil, beispielsweise erhältlich unter der Bezeichnung Pionier 2076 von der Gesellschaft Tudapetrol,
- Mineral Oil, beispielsweise erhältlich unter der Bezeichnung Pionier 6301 von der Gesellschaft Tudapetrol,
- Polydimethylsiloxan, beispielsweise erhältlich unter der Bezeichnung Wacker Silikonöl AK 35 von der Gesellschaft Wacker,
- Isoeikosan, beispielsweise erhältlich unter der Bezeichnung Isoeikosan von der Gesellschaft EC Erdölchemie GmbH,
- Polydimethylsiloxan, beispielsweise erhältlich unter der Bezeichnung Wacker Silikonöl AK 20 von der Gesellschaft Wacker,
- Isofol 1212 Carbonat von der Gesellschaft Condea Chemie,
- Ethoxydiglycol Oleate, beispielsweise erhältlich unter der Bezeichnung Softcutol O von der Gesellschaft Gattefossé,
- Decyl Olivate, beispielsweise erhältlich unter der Bezeichnung Lipodermanol OL von der Gesellschaft Creaderm,
- Dioctylcyclohexane, beispielsweise erhältlich unter der Bezeichnung Cetiol S von der Gesellschaft Henkel,
- Mineral Oil, beispielsweise erhältlich unter der Bezeichnung Pionier 2071 von der Gesellschaft Tudapetrol,
- Paraffinum Liquidum, beispielsweise erhältlich unter der Bezeichnung Hydrobrite 1000 PO von der Gesellschaft WITCO BV,
- Isocetyl Palmitate, beispielsweise erhältlich unter der Bezeichnung Tegosoft HP von der Gesellschaft Goldschmidt,
- Isofol Ester 1693 von der Gesellschaft Condea Chemie,
- Isofol Ester 1260 von der Gesellschaft Condea Chemie,
- Cyclopentasiloxan, beispielsweise erhältlich unter der Bezeichnung Dow Corning Fluid 245 von der Gesellschaft Dow Corning,
- Octyl Isostearate, beispielsweise erhältlich unter der Bezeichnung Prisorine 2036 von der Gesellschaft Unichema,
- Dicaprylyl Carbonate, beispielsweise erhältlich unter der Bezeichnung Cetiol CC von der Gesellschaft Henkel Cognis,
- Trimethylhexyl Isononanoate, beispielsweise erhältlich unter der Bezeichnung Dermol 99 von der Gesellschaft ALZO (ROVI),
- 2- Ethylhexyl Isononanoate, beispielsweise erhältlich unter der Bezeichnung Dermol 89 von der Gesellschaft ALZO (ROVI),
- Dicaprylyl Ether, beispielsweise erhältlich unter der Bezeichnung Cetiol OE von der Gesellschaft Henkel Cognis,
- Dihexyl Carbonate,
- Polydecene, beispielsweise erhältlich unter der Bezeichnung Silkflo 366 NF von der Gesellschaft Albemarle S.A.,
- Octyl Cocoate, beispielsweise erhältlich unter der Bezeichnung Estol 1540 EHC von der Gesellschaft Unichema.

Es ist jedoch auch vorteilhaft, Gemische aus höher- und niederpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, ins-besondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl,Palmkernöl und dergleichen mehr.
Esteröle umfassen Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen. Auch beliebige Abmischungen solcher Öl- und Wachskomponenten sind vorteilhaft im Sinne der vorliegenden Erfindung einzusetzen.

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

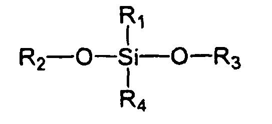

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 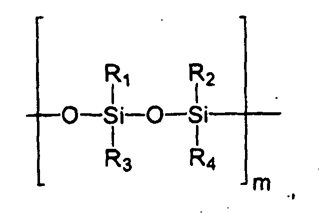 wobei die Siliciumatome mit gleichen oder unterschiedlichen Alkylresten und/oder Arylresten substituiert werden können, welche hier verallgemeinernd durch die Reste R₁ - R₄dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 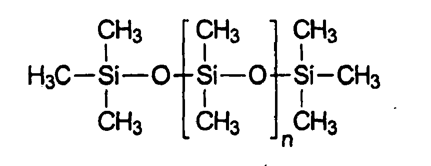 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

| Hersteller | Handelsname | INCI-Name | Polarität |
|---|---|---|---|
| | | | [mN/m] |
| Wacker | Wacker Silikonöl AK 100 | Polydimethylsiloxan | 26,9 |
| Wacker | Wacker Silikonöl AK 50 | Polydimethylsiloxan | 46,5 |
| Wacker | Wacker Silikonöl AK 35 | Polydimethylsiloxan | 42,4 |
| Wacker | Wacker Silikonöl AK 20 | Polydimethylsiloxan | 40,9 |
| Dow Corning | Dow Corning Fluid 245 | Cyclopentasiloxan | 32,3 |
| Dow Corning | Dow Corning Fluid 345 | Cyclomethicone | 28,5 |

Erfindungsgemäß vorteilhaft einzusetzende cyclische Silicone werden im allgemeinen durch Strukturelemente charakterisiert, wie folgt 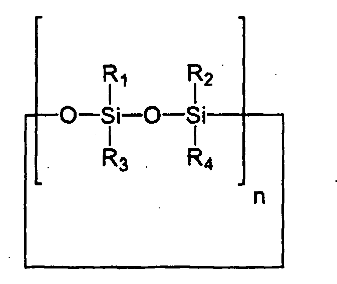 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxanpolyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Es ist erfindungsgemäß bevorzugt, den erfindungsgemäß verwendeten Wirkstoffkombinationen bzw. kosmetischen oder dermatologischen Zubereitungen, solche Wirkstoffkombinationen enthaltend Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,001 Gew.-% bis 10 Gew.-%, bevorzugt zu 0,01 Gew.-% bis 5 Gew.-%, insbesondere bevorzugt zu 0,05 - 2,0 Gew.-%, bezogen auf das Gesamtgewicht der Zubereitungen, enthalten.

Sofern als Emulgatoren nicht die als bevorzugt gekennzeichneten allein zum Einsatz gelangen, können diese zusätzlich oder alternativ gewählt werden aus der Gruppe der nichtionischen oder amphoteren Emulgatoren, die die Funktion als Stabilisatoren einnehmen.

Unter den nichtionischen Emulgatoren befinden sich
a) Ethoxylierte Fettalkohole und Fettsäuren und deren Derivate
b) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
c) Fettalkohole
d) Ethoxylierte Sorbitanester und deren Derivate

Unter den amphoteren Emulgatoren, die die Funktion als Stabilisatoren einnehmen, befinden sich
a) Betaine
b) Sorbitolpolyoxyethylenderivate
Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe derpolyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙR',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙR',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 8 bis 18, ganz besonders vorteilhaft mit mit HLB-Werten von 9 bis 16, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)-stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(1 6)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearyleiher (Ceteareth-15), Polyethylenglycol-(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)iso-stearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol-(18)oleat, Polyethylenglycol(19)oleat, Polyethylenglycol(20)oleat

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)-glyceryllaurat, Polyethylenglycol(23)glyceryllaurat, Polyethylenglycol(6)glycerylcapratlcaprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B.Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Zur Herstellung der Zubereitungen werden wasserlösliche Komponenten mit Ausnahme der Antitranspirant-Wirkstoffe und Teilmenge von Polyol in Wasser gelöst und auf 75 °C erwärmt. Lipophile Komponenten mit Ausnahme des Silikonemugators Abil Care 85 werden vereinigt und ebenfalls auf 75 °C erwärmt. Beide Phasen werden unter Rühren vermischt und gekühlt. Bei 45 °C werden Abil Care 85 und die Restmenge von Polyol zugegeben. Bei 40 °C werden die Antitranspirant-Wirkstoffe und gegebenenfalls noch weitere flüchtige Bestandteile zugegeben und anschließend homogenisiert. Man erhält eine stabile Emulsion.

### Beispiele

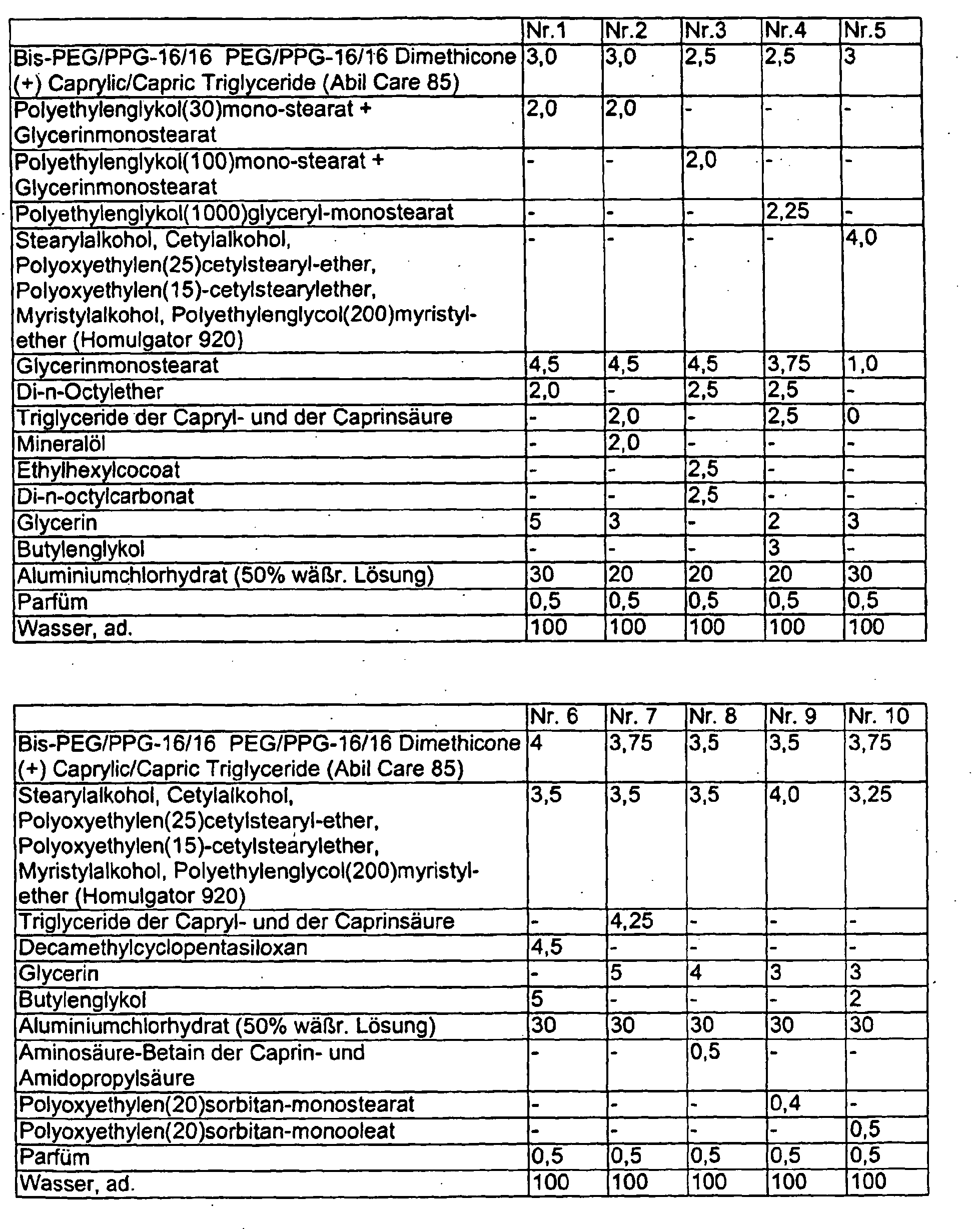

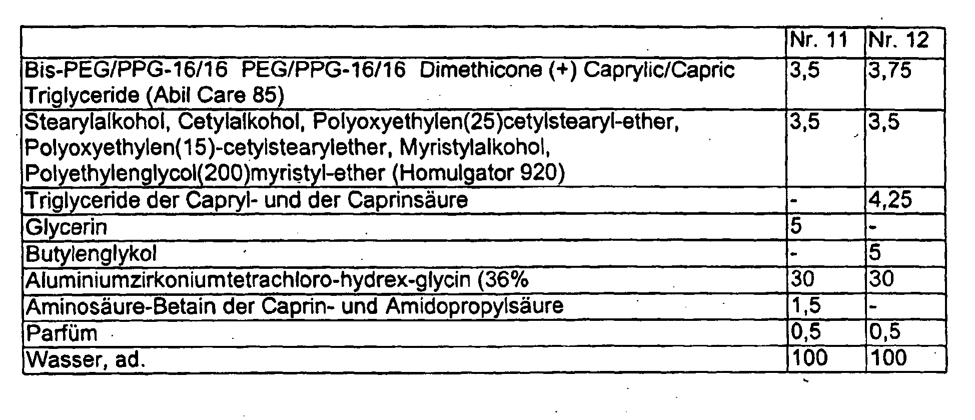

## Patentansprüche

1. Kosmetische und/oder dermatologische Zubereitung in Form von Emulsionen, besonders bevorzugt O/W-Emulsionen, **dadurch gekennzeichnet, daß**
(a) sie Antitranspirantien enthält,
(b) lineare Polydimethylsiloxane R²-(Si(CH₃)₂O)ₙ₋₁-Si(CH₃)₂-R², enthalten sind,
(c) Polydimethylsiloxane R²-(Si(CH₃)₂O)ₙ₋₂-Si(CH₃)R²O-Si(CH₃)₂-R², enthalten sind,
(d) wobei
(d1) R² die Gruppierung -(CH₂)ᵢ-(O-CH₂-CH₂)ₖ-(O-C₃H₈)ᵣ-OH repräsentiert,
(d2) wobei i 2 bis 4 , k und I 12 bis 20 betragen,
(d3) n Werte von 60-100 aufweist und
(d4) die Polydimethylsiloxane (b) und (c) im Verhältnis 80:120 bis 120:80 stehen,
(e) der Anteil der Emulgatoren weniger als 10 Gew.%, besonders bevorzugt weniger als 8 Gew.%, ganz besonders bevorzugt weniger als 5 Gew.% beträgt.

2. Kosmetische und/oder dermatologische Zubereitung in Form von Emulsionen nach Anspruch 1 **dadurch gekennzeichnet, daß** sie einen Gehalt an nichtionischen Coemulgatoren gewählt aus der Gruppe der
Fettalkoholethoxylate oder Gemischen derselbigen, wobei im Falle der Verwendung von Fettalkoholethoxylat-Gemischen eine Komponente eine Kettenlänge von weniger als 14 Kohlenstoffatomen und die andere Komponente eine Kettenlänge von mehr als 16 Kohlenstoffatomen besitzt, bevorzugt Cetylstearylalkohol,
der Fettsäureethoxylate,
der Fettsäuremonoglycerinester, deren Fettsäurekomponente höchstens 24 oder wenigstens 8 Kohlenstoffatome enthält,
der Fettalkohole,
der ethoxylierten Sorbitanester mit einem Ethoxylierungsgrad von 3 bis 100
aufweist.

3. Kosmetische und/oder dermatologische Zubereitung in Form von Emulsionen nach einem der vorangehenden Ansprüche **dadurch gekennzeichnet, daß** im Mittel
i 3 , k und I 16, n 80 betragen und
die Polydimethylsiloxane (b) und (c) im Verhältnis 1:1 stehen.

4. Kosmetische und/oder dermatologische Zubereitung nach einem der vorangehenden Ansprüche **dadurch gekennzeichnet, dass** die Ölphase Ölkomponenten enthält, die aus folgenden Gruppen gewählt werden:
Ester aus einwertigen und/oder mehrwertigen Fettalkoholen mit gesättigten und/oder ungesättigten, verzweigten und/oder unverzweigten Alkancarbonsäuren einer Kettenlänge von 12 bis 25 Kohlenstoffatomen,
gesättigten und/oder ungesättigten, verzweigten und/oder unverzweigten Fettalkoholen einer Kettenlänge von 12 bis 25 Kohlenstoffatomen,
cyclischen oder linearen, gesättigten und/oder ungesättigten, verzweigten und/oder unverzweigten Silikonölen,
Fettsäure-Triglyceride, namentlich Triglycerinester gesättigter und/oder ungesättigter, verzweigter und/oder unverzweigter Alkansäuren einer Kettenlänge von 8 bis 24, insbesondere 12 bis 18 Atomen,
sowie der Mineralöle.

5. Kosmetische und/oder dermatologische Zubereitung nach einem der vorangehenden Ansprüche **dadurch gekennzeichnet, dass** der Anteil der Ölkomponenten kleiner als 15 Gew.%, besonders bevorzugt kleiner als 10Gew.% ist.

6. Kosmetische und/oder dermatologische Zubereitung nach einem der vorangehenden Ansprüche **dadurch gekennzeichnet, dass** sie einen Stabilisator gewählt aus der Gruppe der Sorbitolpolyoxyethylenderivate und/oder der Betaine enthält.

7. Kosmetische und/oder dermatologische Zubereitung nach mindestens einem der vorangehenden Ansprüche **dadurch gekennzeichnet, dass** sie 5 bis 40 Gew.% Antitranspirantien enthält.

8. Kosmetische und/oder dermatologische Zubereitung nach mindestens einem der vorangehenden Ansprüche **dadurch gekennzeichnet, dass** sie 10 bis 25 Gew.% Antitranspirantien enthält.

9. Verwendung von kosmetischen und/oder dermatologischen Zubereitungen nach mindestens einem der vorangehenden Ansprüche als Mittel zur Hemmung der Schweißabsonderung des Menschen.

## Claims

1. Cosmetic and/or dermatological preparation in the form of emulsions, particularly preferably O/W emulsions, **characterized in that**
(a) it comprises antiperspirants,
(b) linear polydimethylsiloxanes R²-(Si(CH₃)₂O)ₙ₋₁-Si(CH₃)₂-R² are present,
(c) polydimethylsiloxanes R²-(Si(CH₃)₂O)ₙ₋₂-Si(CH₃)R²O-Si(CH₃)₂-R² are present,
(d) where
(d1) R² represents the group -(CH₂)ᵢ-(O-CH₂-CH₂)ₖ-(O-C₃H₆)ₗ-OH,
(d2) where i is 2 to 4, k and 1 are 12 to 20,
(d3) n has values from 60-100 and
(d4) the polydimethylsiloxanes (b) and (c) are in the ratio 80:120 to 120:80,
(e) the fraction of the emulsifiers is less than 10% by weight, particularly preferably less than 8% by weight, very particularly preferably less than 5% by weight.

2. Cosmetic and/or dermatological preparation in the form of emulsions according to Claim 1, **characterized in that** it has a content of nonionic coemulsifiers selected from the group of
fatty alcohol ethoxylates or mixtures thereof, where, in the case of the use of fatty alcohol ethoxylate mixtures, one component has a chain length of less than 14 carbon atoms and the other component has a chain length of more than 16 carbon atoms, preferably cetylstearyl alcohol,
the fatty acid ethoxylates,
the fatty acid monoglycerol esters whose fatty acid component comprises at most 24 or at least 8 carbon atoms,
the fatty alcohols,
the ethoxylated sorbitan esters with a degree of ethoxylation of from 3 to 100.

3. Cosmetic and/or dermatological preparation in the form of emulsions according to one of the preceding claims, **characterized in that**, on average,
i is 3, k and 1 are 16, n is 80 and
the polydimethylsiloxanes (b) and (c) are in the ratio 1:1.

4. Cosmetic and/or dermatological preparation according to one of the preceding claims, **characterized in that** the oil phase comprises oil components which are selected from the following groups:
esters of monohydric and/or polyhydric fatty alcohols with saturated and/or unsaturated, branched and/or unbranched alkanecarboxylic acids of chain length from 12 to 25 carbon atoms,
saturated and/or unsaturated, branched and/or unbranched fatty alcohols of chain length from 12 to 25 carbon atoms,
cyclic or linear, saturated and/or unsaturated, branched and/or unbranched silicone oils,
fatty acid triglycerides, mainly triglycerol esters of saturated and/or unsaturated, branched and/or unbranched alkanoic acids of chain length from 8 to 24, in particular 12 to 18 atoms,
and also the mineral oils.

5. Cosmetic and/or dermatological preparation according to one of the preceding claims, **characterized in that** the fraction of the oil components is less than 15% by weight, particularly preferably less than 10% by weight.

6. Cosmetic and/or dermatological preparation according to one of the preceding claims, **characterized in that** it comprises a stabilizer selected from the group of sorbitol polyoxyethylene derivatives and/or the betaines.

7. Cosmetic and/or dermatological preparation according to at least one of the preceding claims, **characterized in that** it comprises 5 to 40% by weight of antiperspirants.

8. Cosmetic and/or dermatological preparation according to at least one of the preceding claims, **characterized in that** it comprises 10 to 25% by weight or antiperspirants.

9. Use of cosmetic and/or dermatological preparations according to at least one of the preceding claims as compositions for inhibiting the secretion of perspiration in people.

## Revendications

1. Préparation cosmétique et/ou dermatologique sous forme d'émulsions, de manière particulièrement préférée d'émulsions huile-dans-eau, **caractérisée en ce que**
(a) elle contient des antiperspirants,
(b) elle contient des polydiméthylsiloxanes linéaires R²-(Si(CH₃)₂O)ₙ₋₁-Si(CH₃)₂-R²,
(c) elle contient des polydiméthylsiloxanes R²-(Si(CH₃)₂O)ₙ₋₂-Si(CH₃)R²O-Si(CH₃)₂-R²,
(d) où
(d1) R² représente le groupement -(CH₂)ᵢ-(O-CH₂-CH₂)ₖ-(O-C₃H₆)ₗ-OH,
(d2) i valant 2 à 4, k et 1 valant 12 à 20,
(d3) n présente des valeurs de 60-100 et
(d4) les polydiméthylsiloxanes (b) et (c) se trouvent dans un rapport de 80:120 à 120:80,
(e) la proportion des émulsifiants est inférieure à 10% en poids, de manière particulièrement préférée inférieure à 8% en poids, de manière tout particulièrement préférée inférieure à 5% en poids.

2. Préparation cosmétique et/ou dermatologique sous forme d'émulsions selon la revendication 1, **caractérisée en ce qu'**elle présente une teneur en co-émulsifiants non ioniques choisis dans le groupe formé par
- les éthoxylates d'alcools gras ou des mélanges de ceux-ci, où, dans le cas de l'utilisation de mélanges d'éthoxylates d'alcools gras, un composant présente une longueur de chaîne inférieure à 14 atomes de carbone et l'autre composant présente une longueur de chaîne supérieure à 16 atomes de carbone, de préférence l'alcool cétylstéarylique,
- les éthoxylates d'acides gras,
- les esters monoglycéroliques d'acides gras, dont le composant acide gras contient au plus 24 ou au moins 8 atomes de carbone,
- les alcools gras,
- les esters éthoxylés de sorbitane, présentant un degré d'éthoxylation de 3 à 100.

3. Préparation cosmétique et/ou dermatologique sous forme d'émulsions selon l'une quelconque des revendications précédentes, **caractérisée en ce qu'**en moyenne i vaut 3, k et 1 valent 16, n vaut 80 et les polydiméthylsiloxanes (b) et (c) se trouvent dans un rapport de 1:1.

4. Préparation cosmétique et/ou dermatologique selon l'une quelconque des revendications précédentes, **caractérisée en ce que** la phase huileuse contient des composants huileux qui sont choisis dans les groupes suivants :
- les esters d'alcools gras monovalents et/ou polyvalents avec des acides alcanecarboxyliques saturés et/ou insaturés, ramifiés et/ou non ramifiés présentant une longueur de chaîne de 12 à 25 atomes de carbone.
- les alcools gras saturés et/ou insaturés, ramifiés et/ou non ramifiés présentant une longueur de chaîne de 12 à 25 atomes de carbone,
- les huiles de silicone cycliques ou linéaires, saturées et/ou insaturées, ramifiées et/ou non ramifiées,
- les triglycérides d'acides gras, notamment les esters de triglycérol d'acides alcanoiques saturés et/ou insaturés, ramifiés et/ou non ramifiés, présentant une longueur de chaîne de 8 à 24, en particulier de 12 à 18 atomes,
- ainsi que les huiles minérales.

5. Préparation cosmétique et/ou dermatologique selon l'une quelconque des revendications précédentes, **caractérisée en ce que** la proportion de composants huileux est inférieure à 15% en poids, de manière particulièrement préférée inférieure à 10% en poids.

6. Préparation cosmétique et/ou dermatologique selon l'une quelconque des revendications précédentes, **caractérisée en ce qu'**elle contient un stabilisateur choisi dans le groupe des dérivés de sorbitol-polyoxyéthylène et/ou des bétaïnes.

7. Préparation cosmétique et/ou dermatologique selon au moins l'une quelconque des revendications précédentes, **caractérisée en ce qu'**elle contient 5 à 40% en poids d'antiperspirants.

8. Préparation cosmétique et/ou dermatologique selon au moins l'une quelconque des revendications précédentes, **caractérisée en ce qu'**elle contient 10 à 25% en poids d'antiperspirants.

9. Utilisation de préparations cosmétiques et/ou dermatologiques selon au moins l'une quelconque des revendications précédentes, comme agent pour inhiber la sécrétion de sueur chez l'homme.
